# EUROPÄISCHE PATENTSCHRIFT

(11) **EP 0 992 577 B1**
(45) Veröffentlichungstag und Bekanntmachung des Hinweises auf die Patenterteilung: **26.07.2000**
(21) Anmeldenummer: 98110294.0
(22) Anmeldetag: 05.06.1998
(51) Int. Cl.: C12M 3/00, B01L 3/02, C12N 15/89

(54) **Mikroinjektionsverfahren zum Einbringen eines Injektionsstoffes, insbes. fremdes, genetisches Material, in Prokaryoten-und Eukaryotenzellen, sowie Zellkompartimente von letzteren (Plastiden, Zellkerne), sowie Nanopipette hierzu**
Process for microinjection and nanopipette for introducing an injection product, particularly foreign genetic material in procaryotic or eucaryotic cells or cellcomparments thereof (plastides, cell nucleus)
Procédé de microinjection et nanopipettes pour l'introduction d'un produit d'injection, plus particulièrement un materiau génétique étranger dans des cellules procaryotes ou eucaryotes ou dans desc compartiments cellulaires de celles-ci (plastides, noyau cellulaire)

(43) Veröffentlichungstag der Anmeldung: 12.04.2000
(73) Patentinhaber: Lummel, Wolfgang, 6300 Zug (CH)
(72) Erfinder: Knoblauch, Michael, 35510 Butzbach (DE)
(74) Vertreter: von Raffay, Vincenz, Dipl.-Ing.

(56) Entgegenhaltungen:
- EP-A- 0 292 899
- DE-A- 4 423 267
- DE-A- 19 629 143
- DE-U- 29 801 523
- GB-A- 2 211 111
- US-A- 3 720 354
- US-A- 4 625 677
- US-A- 5 225 750
- PATENT ABSTRACTS OF JAPAN vol. 017, no. 509 (P-1612), 13. September 1993 (1993-09-13) & JP 05 133851 A (NAGANO JAPAN RADIO CO), 28. Mai 1993 (1993-05-28)

## Beschreibung

Die vorliegende Erfindung betrifft ein Mikroinjektionsverfahren zum Einbringen eines Injektionsstoffes, insbesondere fremdes, genetisches Material, in Prokaryoten- und Eukaryotenzellen, sowie Zellkompartimenten von letzteren (Plastiden, Zellkerne) nach dem Oberbegriff des Anspruchs 1, sowie eine Nanopipette zur Durchführung dieses Verfahrens.

Der Stand der Technik stellt sich wie folgt dar:

Intrazelluläre Mikroinjektion von Fluoreszenzfarbstoffen (Kempers & van Bel 1997), Antikörpern (Kamei et al, 1996; Oka et al, 1990; Höner et al, 1988), anderen Proteinen (Rose et al, 1992; Staiger et al, 1994;Walton et al, 1992) und genetischem Material (Kost et al, 1995; Nguyen et al, 1996; Heinzel et al, 1997) ist eine häufig verwendete Methode, trotz etlicher Nachteile die mit dem Einstechen einer Mikroelektrode in eine Zelle verbunden sind. Die offensichtlichsten Nachteile der Technik sind mit der Verletzung (Verlust von Cytoplasma) der Zelle durch die Glasspipette verbunden.

Damit die Zelle nicht irreparabel zerstört wird, muss sich die Plasmamembran, die die Zelle umgibt, um die eingestochene Spitze der Pipette schließen damit ein Auslaufen des Zellinhaltes verhindert wird. Gerade dieses Problem tritt aber häufig beim Einstechen von Mikropipetten (Spitzendurchmesser 0.5-1 µm) in kleine Zellen (Durchmesser 10-20 µm) auf. Darüber hinaus besitzen viele Zellen einen hohen Innendruck, den Turgor (alle Pflanzenzellen bis 4MPa [ 40 bar] Prokaryotenzellen und einige Tierzellen), der die Probleme deutlich verschärft. Nach Einstechen entlädt sich der Druck um die Pipette und ebenso über die Pipettenspitze in das Innere der Pipette. Das letztere Phänomen wird durch das sogenannte "backfiring" des Elektrodeninhalts sichtbar (Van der Schoot, 1989). Drastische ultrastrukturelle Änderungen durch das Einstechen von Pipetten (Spitzendurchmesser von 1µm und größer) konnten z.B. bei Siebelementen (pflanzliches Leitgewebe) gezeigt werden (Knoblauch und Van Bel 1998). Bei Pipetten mit einem Spitzendurchmesser von 0.1 µm zeigte sich dieses Phänomen jedoch nicht. Hieraus resultierte das Ziel, die Spitze der Pipette so klein zu machen, daß die Einströmgeschwindigkeit des Zellinhaltes in die Pipette geringer als die Wasseraufnahmefähigkeit der Zelle über ihre eigene Membran wird. D.h., daß die Zelle ihren Innendruck durch Wasseraufnahme aufrechterhalten kann, um so das Phänomen des backfirings zu verhindern. Zusätzlich würde eine solche Pipette (Spitzendurchmesser ca.0.1µm, also 25 bis 100 mal kleiner als konventionelle) ein viel kleineres Loch in der Plasmamembran der Zelle verursachen und damit die Probleme des Auslaufens um die Einstichstelle minimieren. Ein gutes "sealing" ist auch bei sehr kleinen Zellen zu erwarten.

Auch in der DE-C2-37 38 874 wird eine Mikronadel mit einem Spitzendurchmesser von ca. 1 µm verwendet, um ein Verfahren zur Herstellung von genetisch transformierten Pflanzenobjekten durchzuführen, und zwar durch Einführung eines transformierenden Faktors in das Rezipientenobjekt, und zwar in einen pflanzlichen Protoplasten und nachfolgende Selektion von Zellinien und Pflanzen aus diesem Rezipientenobjekt, die neue erbliche Eigenschaften aufweisen, wobei als transformierender Faktor Makromoleküle verwendet werden und der transformierende Faktor in das Rezipientenobjekt durch Mikroinjizierung einführt, das dadurch gekennzeichnet ist, daß man als transformierenden Faktor entweder ein DNS-Molekül oder ein autonom replizierendes Organell und als Rezipientenobjekt entweder eine Einzelzelle oder eine Zelle im Zellverband verwendet.

Die negative Konsequenz einer solch kleinen Spitze ist jedoch der sehr hohe Druck der benötigt wird, um durch die winzige Öffnung noch Stoffe in eine Zelle injizieren zu können. Dafür sind die herkömmlichen Druckinjektionsgeräte nicht geeignet. Sie können den benötigten Druck nicht aufbauen, um in einem für eine Injektion vernünftigen Zeitraum (max. wenige Minuten) genügend Material in die Zelle zu pressen.

Der Erfindung liegt deshalb die Aufgabe zugrunde, das eingangs genannte Verfahren bzw. die Nanopipette derart zu verbessern, daß es bzw. sie die geschilderten Nachteile sicher vermeidet und ein leichtes, schnelles und zerstörungsfreies Einbringen von Injektionsstoff in Prokaryoten- und Eukaryotenzellen sowie Zellkompartimenten von letzteren, ermöglicht.

Diese Aufgabe wird durch das im Anspruch 1 gekennzeichnete Verfahren bzw. durch die neu gestaltete Nanopipette und ihren Inhalt gemäß Anspruch 9 gelöst.

Es hatte sich also gezeigt, daß man in vorher nicht erreichbare Kompartimente oder Prokaryoten einstechen kann, ohne daß ein Verlust von Cytoplasma derselben auftritt.

Die erfindungsgemäße Nanopipette basiert auf der Grundlage, daß die wärmeinduzierte Expansion eines Stoffes oder Stoffgemisches, mit dem die Pipette gefüllt ist, den zu injizierenden Stoff aus der Pipettenspitze drückt, die einen Spitzendurchmesser von nur 0,025 - 0,3 µm, insbesondere 0,05 - 0,2 µm aufweist, wobei je nach Füllstoff oder -gemisch stufenweise gewünschte Drucke aufgebaut werden können.

Grundsätzlich können verschiedenste Flüssigkeiten oder Feststoffe als Füllstoff oder -gemisch dienen. Entscheidend für die Anwendbarkeit eines Stoffes oder -gemisches ist sein Ausdehnungskoeffizient im Verhältnis zur Ausdehnung der Glaswände der Nanopipette, da sich diese bei Erwärmung ebenfalls ausdehnen. Versuche haben ergeben, daß Ausdehnungskoeffizienten im Bereich derer von Metallen am idealsten sind. Zur Befüllung kommen im Bereich der Metalle jedoch nur niedrigschmelzende Legierungen oder reine Metalle in Frage (max bis +200°C), da der Füllstoff im flüssigen Zustand eingefüllt werden muss. Bei höheren Temperaturen würden die Glaswandungen der Pipette weich werden und die winzige Spitze zerfließen. Quecksilber wäre deshalb ein idealer Kandidat, da es bei Raumtemperatur bereits flüssig ist. Es hat jedoch den unumgänglichen Nachteil, daß es für lebende Zellen hoch toxisch ist und deshalb nicht in Frage kommt. Darüber hinaus darf es in offenen Systemen nicht verwendet werden. Andere niederigschmelzende Legierungen mit einem Schmelzpunkt zwischen +50 und +200 °C (Z.B. Blei-Zinn Eutektikum) können nach Schmelzen in die Pipette eingefüllt werden. Beim Erkalten löst sich das Metall von der Glaswand. Der winzige Spalt, der dabei zwischen Metall und Glaswand entsteht und bis in die Spitze reicht kann anschließend mit dem zu injizierenden Stoff befüllt werden. Nach druckdichtem Verschliessen der Rückseite und erneutem langsamen Erwärmen beginnt das Metall sich wieder auszudehnen und preßt den Injektionsstoff durch die Spitze. Der Nachteil dieser Verfahrensweise ist, daß sich das Metall beim Erkalten meist unregelmäßig von der Wand löst und kleine Kristalle bildet. Dadurch ist ein blasenfreies Befüllen der Elektrode kaum möglich. Das führt dazu, daß durch die hohe Komprimierbarkeit der restlichen Gasblasen der Druck deutlich langsamer steigt. Es soll aber darauf hingewiesen werden, daß ein solches System durchaus im Sinn der Erfindung funktionstüchtig ist.

Die oben beschriebenen Probleme sind bei Stoffen, die bei Zimmertemperatur flüssig sind, nicht vorhanden. Die Pipette kann bei Zimmertemperatur befüllt werden und es treten keine Unregelmäßigkeiten in der Oberfläche und keine Kristallisationserscheinungen auf. Dadurch sind solche Stoffe grundsätzlich vorzuziehen. Wie oben erwähnt ist Quecksilber jedoch das einzige reine Metall das bei Zimmertemperatur flüssig ist, das wiederum durch seine Toxizität nicht einsetzbar ist. Andere organische Flüssigkeiten wie Silikonöl etc. haben einen zu hohen Ausdehnungskoeffizienten. In Versuchen mit reinem Silikonöl reichte der Atem oder die Körperwärmestrahlung der den Versuch durchführenden Person vor dem Mikroskop aus, um einen massiven Ausstrom aus der Pipettenspitze hervorzurufen. Ein solches System ist viel zu temperatursensibel, um es vernünftig steuern zu können.

Als idealer, bevorzugter Füllstoff erwies sich jedoch eine neu entwickelte Gallium-Indium-Zinn Legierung (Galinstan, Marke der Geraberger Thermometerwerke, Geschwenda, DE [vgl. EP-B1- 0 657 023]). Diese Metallegierung ist bis - 20°C flüssig. Sie ist nach dem heutigen Stand der Wissenschaft völlig ungiftig und besitzt mit 11.5γ 10⁻⁵ K⁻¹ einen Raumausdehnungskoeffizienten, der gegenüber dem des Quecksilbers (18.1γ 10⁻⁵ K⁻¹) bei ca. 63 % liegt. Der geringe Ausdehnungskoeffizient führt dazu, daß bei einer reinen Verwendung von Galinstan der Druck sehr langsam aufgebaut wird. Wichtig für biologische Proben ist jedoch, daß bestimmte Temperaturbereiche nicht über- oder unterschritten werden. Eine zu hohe Sensibilität wie die des Silikonöls führt dazu, daß die Pipette nicht entsprechend gesteuert werden kann. Eine zu geringe Sensibilität hingegen führt dazu, daß die Pipette für biologische Proben zu stark erhitzt werden muss, um den nötigen Druck aufzubauen. Die Proben könnten beschädigt werden. Deshalb wird bevorzugt eine Kombination von Galinstan mit Silikonöl verwendet. Dieses 2 Komponenten-Füllstoffgemisch vereinigt die starke Ausdehnung von Silikonöl mit der schwachen des Galinstans. D.h. je mehr Silikonöl in Relation zu Galinstan verwendet wird, desto wärmesensibler wird die Pipette. Dies macht die Pipette sehr variabel und man kann Sie optimal an alle Bedingungen für die verschiedensten Injektionen anpassen, die sich bei biologischen Proben im Bereich von 5-10°C Temperaturänderung abspielen. Vorteilhafterweise ist bei einer Borsilikatglasnanopipette das Mengenverhältnis von Galinstan zu Silikonöl wie 4 : 1 Teile.

Grundsätzlich können zur Herstellung der Glaspipetten alle Glastypen verwendet werden, die auch normalerweise zur Herstellung von Pipetten oder Mikroelektroden verwendet werden (z.B. Borosilikatglas, Quarzglas). Für bestimmte Anwendungen ist es aber von Vorteil, wenn spezielle Glastypen verwendet werden. Beispielsweise ist beim Einstechen in kleine Zellkompartimente (Chloroplasten etc.) und dem anschliessenden Erwärmen der Pipette durch das Ausdehnen des Glases eine Wanderung durch das Kompartiment zu beobachten. In solchen Fällen ist es von Vorteil Quarzglas zu verwenden, da dieses einen 10-20fach geringeren Ausdehnungskoeffizienten besitzt als andere Glastypen.

Die Befüllung der Pipette wird wie folgt durchgeführt. Es werden Kapillaren mit innerem Filament verwendet. Nachdem die Kapillare zur Pipette ausgezogen ist, wird die Spitze zuerst mit dem zu injizierenden Stoff mittels der am Filament wirkenden Kapillarkräfte befüllt. Anschließend folgen Galinstan und Silikonöl in den entsprechenden Mengen. Nachdem die Pipette vollkommen und blasenfrei befüllt ist, wird sie am Ende druckdicht verschlossen. Das Verschließen erfolgt vorteilhafterweise mit Hilfe einer ca. 1 cm langen Glaskappe, deren Innendurchmesser geringfügig größer ist als der Außendurchmesser der Pipette. Die Glaskappe wird mit einem 2 Komponentenkleber befüllt und über die Rückseite der Pipette gestülpt. Nach Aushärten des Klebers ist die Pipette durch den geringen Spalt zwischen Kappe und Pipette sowie die in Längsrichtung auf den Kleber wirkenden Scherkräfte druckdicht verschlossen. In Fällen, in denen mit extrem hohen Drucken gearbeitet wird, kann die Rückseite der Pipette ebenfalls zu einer Spitze ausgezogen werden. Da der Druck pro Fläche wirkt, verringert sich durch die Verkleinerung der Fläche der auf die Kappe wirkende Druck. Somit wird ein druckdichter Verschluss auch bei Maximaldrucken sichergestellt. Der Kleber sollte stets ein 2 Komponenten- oder Polymerisationskleber sein, da diese beim Aushärten nur geringfügig schrumpfen. Hingegen zeigen Kleber mit Lösungsmittel eine deutlich stärkere Schrumpfung, da das Lösungsmittel sich verflüchtigt.

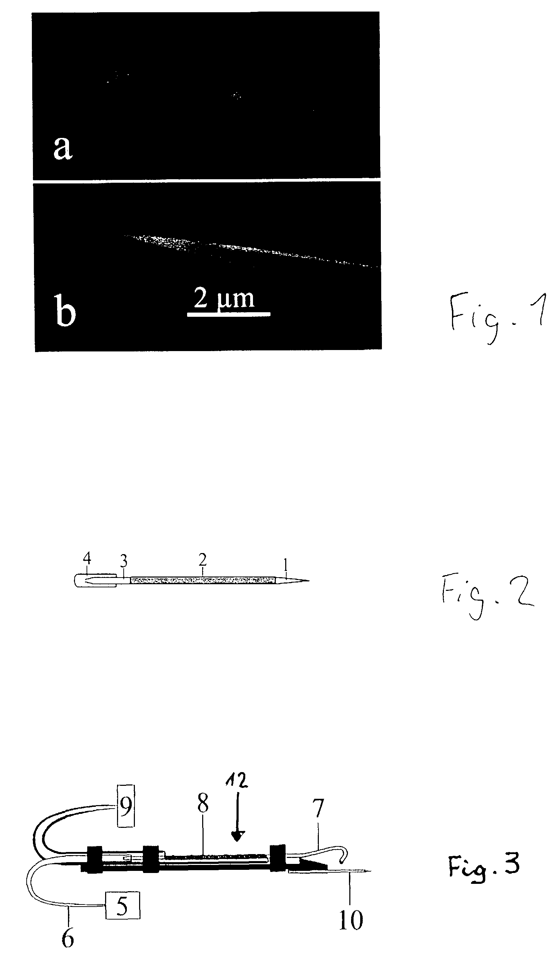

Als Heizvorrichtung sind grundsätzlich verschiedene Möglichkeiten denkbar. Wichtig ist, daß die Heizvorrichtung von der Pipette entkoppelt ist, um Vibrationen zu verhindern. Vorteilhafterweise wird ein von einer Pumpe angetriebener Luftstrom über einen Schlauch und eine Glaskapillare auf die Pipette geblasen (Abbildung 3). Der Luftstrom ist vom Präparat weggerichtet. Um die Glaskapillare ist ein Konstantandraht (Heizdraht) gewickelt, der mit einem stufenlos regulierbaren Netzteil verbunden ist. Durch Erwärmen des Heizdrahtes kommt es zur Erwärmung des Luftstromes und letztendlich zum Erwärmen der Pipette.

Da die Pipette mit Metall befüllt ist, könnten aber auch Induktionsheizungen etc. verwendet werden.

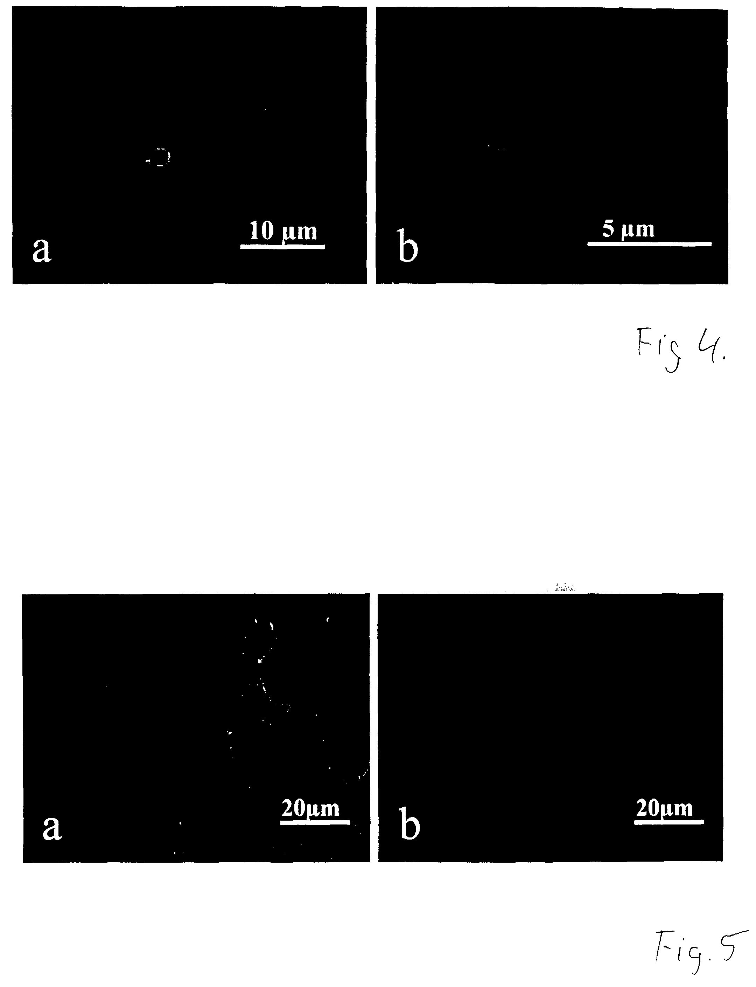

Die Vorteile der erfindungsgemäßen Pipette sind vielfältig und beinhalten die Möglichkeit der Injektion in Prokaryoten (Bakterien etc., Abbildung 4a), Plastiden wie Chloroplasten (Abbildung 4b), Zellkerne (Abbildung 5a,b) etc.. Zellkerninjektionen werden bei tierischen Zellen bereits routinemäßig mittels Pipetten mit Spitzendurchmessern von 0.5-1 µm durchgeführt. Die neue temperaturgesteuerte Nanopipette hat jedoch eine 25-100 mal geringere Spitzenfläche als die herkömmlichen Pipetten wodurch deutlich geringere Verletzungen zu erwarten sind. Erstmals können auch Zellkerne oder Zellkompartimente in hochturgeszenten Pflanzen- und Tierzellen erreicht werden (Abbildung 4b) ohne einen Druckverlust in der Zelle zu verursachen. Die Möglichkeiten der Mikroinjektion stehen damit erstmals auch den Mikrobiologen zur Verfügung. Die Injektion von Makromolekülen, die für bestimmte Bereiche der Genetik, sowie der Zell- und Medizinischen Forschung essentiell sind, kann mit geringerer Verletzung der Zellen und durch Injektion geringerer Mengen durchgeführt werden. Allgemein ist eine präzisere Arbeit möglich. Des weiteren ergibt sich durch das Vorheizen der Pipette und dem Abkühlen nach Einstechen die Möglichkeit der Entnahme von Zellsäften oder Säften aus Kompartimenten.

Die Pipette ist extrem variabel zu gestalten. Sie kann durch
(a)Veränderung des inneren Durchmessers der Glaskapillare,
(b) Veränderung des Verhältnisses der Füllstoffe, (c) Anpassung des Spitzendurchmessers, (d) Verwendung verschiedener Glastypen etc. jeglichen experimentellen Erfordernissen angepasst werden.

Im folgenden werden bevorzugte Ausführungsbeispiele der Erfindung anhand der Zeichnung näher beschrieben.

Es zeigt:
- Fig. 1a:: Rasterelektronenmikroskopische Aufnahme der vorderen Teils einer konventionellen Pipette (Spitzendurchmesser ca 0.7 µm) und
- Fig. 1b:: einer thermogesteuerten erfindungsgemäßen Nanopipette (Spitzendurchmesser ca. 0.1 µm),
- Fig. 2:: eine schematische Darstellung einer erfindungsgemäß fertig befüllten thermogesteuerten Nanopipette 10. Nach Befüllen der Nanopipette (10)mit Injektionsstoff (1), Galinstan (2) und Silikonöl (3) wird die Kapillare mit einer mit Kleber befüllten Glaskappe (4) verschlossen,
- Fig. 3:: eine schematische Darstellung der erfindungsgemäßen Heizvorrichtung: ein durch eine Pumpe (5) produzierter Luftstrom wird über einen Schlauch (6) und eine Glaskapillare (7) auf die Pipette (10) geblasen. Die Luft wird über einen um die Glaskapillare (7) gewundenen Konstantandraht (8), der mit einem stufenlos regelbaren Netzteil verbunden ist, erwärmt,
- Fig. 4:: Confocale Laser Scanning mikroskopische Aufnahmen von Mikroinjektionen mit der erfindungsgemäßen thermogesteuerten Nanopipette. (a) Injektion von Lucifer Yellow (grün = hellste Stelle in der s/w-Abbildung) in eine einzelne Zelle des Cyanobakteriums *Nostoc* muscorum (Prokaryot). Rot (=graue Stellen) ist die Autofluoreszenz des Cholorophylls. (b) Injektion von Lucifer Yellow (grün) in einen einzelnen Chloroplasten einer *Vicia faba* Mesophyllzelle. Die anderen Chloroplasten sind als rot fluoreszierende Körper erkennbar,
- Fig. 5:: Confocale Laser Scanning mikroskopische Aufnahmen von Mikroinjektionen mit der erfindungsgemäßen thermogesteuerten Nanopipette in Kerne der Zellen von Xenopus distale Nierentubus A6 Zellinie Kulturen. (a) Lucifer Yellow (ca.0.5 kDa) wurde in den unteren Kern injiziert und verlässt den Kern schnell über die Kernporen und wird im anderen Kern ebenfalls akkumuliert (b). Ein 70 kDa Dextran-Lucifer Yellow Konjugat verlässt den Kern hingegen nur sehr langsam.

### Literatur

1. Kempers, R. & van Bel, A. J. E. Symplasmic connections between sieve element and companion cell in the stem phloem of *Vicia faba* L. have a molecular exclusion limit of at least 10 kDa Planta 201, 195-201 (1997).
2. Kamei, Y., Xu, L., Heinzel, T., Torchia, J., Kurokawa, R., Gloss, B., Lin, S-C., Heyman. R. A., Rose, D. W., Glass, C. K. & Rosenfeld, M. G. A CBP integrator complex mediates transcriptional activation and AP-1 inhibition by nuclear receptors. Cell 85, 403-414 (1996).
3. Oka, M. T., Arai, T. & Hamaguchi, Y. Microinjection ofthe monoclonal anti-tubulin antibody YL1/2 inhibits cleavage of sand dollar eggs. Cell Structure and Function 15, 373 - 378 (1990).
4. Höner, B., Citi, S.. Kendrick-Jones, J. & Jockusch, B. M. Modulation of cellular morphology and locomotory activity by antibodies against myosin. J. Cell. Biol. 107, 2181-2189 (1988).
5. Rose, D. W., McCabe, G., Fermisco, J. R. & Adler, M. Expression of *c-fos* and AP-1 activity in senescent human fibroblasts is not sufficient for DNA synthesis. J. Cell Biol. 119, 1405-1411 (1992).
6. Staiger, C. J., Yuan, M., Valenta, R., Shaw, P. J., Warn, R. M. & Lloyd. C. W. Microinjected profilin affects cytoplasmic streaming in plant cells by rapidly depolymerizing actin microfilaments. Current Biology 4, 215-219 (1994).
7. Walton, P. A., Gould, S. J., Rachubinski, R. A., Subramani, S. & Feramisco J. R. Transport of microinjected alcohol oxidase from *Pichia pastoris* into vesicles m mammalian cells: involvement of the peroxisomal targeting signal. J. Cell. Biol. 118,499-508 (1992).
8. Kost, B., Galli, A., Potrykus, L & Neuhaus. G. High efficiency transient and stable transformation by optimized DNA microinjection into *Nicotiana tabacum* protoplasts. J. Exp. Bot. 46. 1157-1167 (1995).
9. Nguyen, L.. Lucas, W. J.. Ding, B. & Zaitlin, M. Viral RNA trafficking is inhibited in replicase-mediated resistent transgenic tobacco plants. Proc. Natl. Acad. Sci. USA 93, 12643-12647 (1996).
10. Heinzel, T., Lavinsky, R. M., Mullen, T-M., Söderström, M., Laherty, C. D., Torchia. J., Yang, W-M., Brard. G., Ngo. S. D., Davie, J. R., Seto, E., Eisenmann, R. N., Rose, D. W., Glass. C. K. & Rosenfeld, M. G. A complex containing N-CoR, mSin3 and histone deacetylase mediates transcriptional repression. Nature 387, 43- 48 (1997)
11. Van der Schoot, C. Determinants of xylem-to-phloem transfer in tomato. PhD dissertation. University of Utrecht. (1989)
12. Knoblauch, M. & van Bel, A. J. E. Sieve tubes in action. Plant Cell 10, 35-50 (1998)

## Patentansprüche

1. Mikroinjektionsverfahren zum Einbringen eines Injektionsstoffes, insbesondere fremdes, genetisches Material in Prokaryoten- und Eukaryotenzellen, sowie Zellkompartimente von letzteren (Plastiden, Zellkerne) mittels einer Nanopipette und Druck,
dadurch gekennzeichnet, daß man die Nanopipette (10), welche einen Aussendurchmesser von 0,5 - 2 mm, einen Innendurchmesser von 0,1 - 1,5 mm und einen Spitzendurchmesser von 0,025 - 0,3 µm besitzt, mit dem Injektionsstoff (1) und einem durch Wärme expandierbaren Stoff oder -gemisch füllt und die Kapillare der Nanopipette (10) anschließend mit einem Klebemittel verschließt, die Pipettenspitze mit Hilfe eines Mikroskopes und eines Mikromanipulators in die gewünschte Plastide, das Bakterium oder das Zellkompartiment/-Zellkern einsticht, und die Nanopipette mittels einer regelbaren Heizvorrichtung (12) erwärmt, bis der Injektionsstoff mit einer Ausströmgeschwindigkeit im Bereich bis zu 1 Femtoliter pro Sekunde an der Pipettenspitze aus- und in die Plastide, das Bakterium oder das Zellkompartiment/den Zellkern, deren Durchmesser im Bereich von 1 - 20 µm liegt, eintritt.

2. Mikroinjektionsverfahren nach Anspruch 1, dadurch gekennzeichnet, daß ein durch eine Pumpe (5) produzierter Luftstrom über einen Schlauch (6) und eine Glaskapillare (7) auf die Nanopipette (10) geblasen wird, wobei die Luft über einen um die Glaskapillare (7) gewundenen Konstantandraht (8), der mit einem stufenlos regelbaren Netzteil (9) verbunden ist, erwärmt wird.

3. Verfahren nach Anspruch 1 oder 2, dadurch gekennzeichnet, daß die Nanopipette (10) mittels eines 2-Komponentenklebers und einer Glaskappe (4) verschlossen wird.

4. Verfahren nach Anspruch 1 bis 3, dadurch gekennzeichnet, daß 0,1 - 100 Femtoliter Injektionsstoff innerhalb von 5 Minuten in die Plastide, das Bakterium oder das Zellkompartiment/Zellkern gepreßt wird.

5. Verfahren nach Anspruch 1 bis 4, dadurch gekennzeichnet, daß der wärmeexpandierbare Stoff oder -gemisch ein ungiftiges reines Metall oder Metallegierung mit einem Schmelzpunkt zwischen -20° bis +200°C ist, der bzw. das einen mit Quecksilber vergleichbaren Ausdehnungskoeffizienten besitzt.

6. Verfahren nach Anspruch 5, dadurch gekennzeichnet, daß die ungiftige Metallegierung eine Gallium-Indium-Zinn-Legierung ist, insbesondere Galinstan (Marke der Fa. Geraberger Thermometerwerke, Geschwenda, DE), die bis -20°C flüssig ist und einen Raumausdehnungskoeffizienten von 11,5 y 10⁻⁵K⁻¹ besitzt.

7. Verfahren nach Anspruch 6, dadurch gekennzeichnet, daß Galinstan zusammen mit einem Silikonöl eingesetzt wird.

8. Verfahren nach Anspruch 7, dadurch gekennzeichnet, daß bei einer Borosilikatglaspipette 4 Teile Galinstan und 1 Teil Silikonöl eingesetzt werden.

9. Nanopipette (10) zur Durchführung des Verfahrens nach einem oder mehreren der vorstehenden Ansprüche, gekennzeichnet durch folgende Merkmale: einen Außendurchmesser von 0,5 - 2 mm, einen Innendurchmesser von 0,1 - 1,5 mm und einen Spitzendurchmesser von 0,025 - 0,3 µm besitzt, die durch eine regelbare Heizvorrichtung (12) zur thermogesteuerten Injektion erwärmbar ist.

## Claims

1. Microinjection process for introducing an injection substance, particularly foreign, genetic material, into procaryotic and eucaryotic cells, as well as cell compartments of the latter (plastids, cell nuclei) by means of a nanopipette and pressure, characterized in that the nanopipette (10), which has an external diameter of 0.5 to 2 mm, an internal diameter of 0.1 to 1.5 mm and a tip diameter of 0.025 to 0.3 µm, is filled with the injection substance (1) and a heat-expandable substance or substance mixture and the capillary of the nanopipette (10) is subsequently sealed with an adhesive, the pipette tip being stuck with the aid of a microscope and a micromanipulator into the desired plastids, bacterium or cell compartment/ cell nucleus, and the nanopipette is heated by means of an adjustable heater (12) until the injection substance passes out of the pipette tip at an outflow rate of up to 1 femtolitre per second and enters the plastids, bacterium or cell compartment/cell nucleus, whose diameter is 1 to 20 µm.

2. Microinjection process according to claim 1, characterized in that an air flow produced by a pump (5) is blown by means of a hose and glass capillary (7) onto the nanopipette (10), the air being heated by means of a constantan wire (8) wound round the glass capillary (7) and connected to a continuously variable power supply .

3. Process according to claim 1 or 2, characterized in that the nanopipette (10) is sealed by means of a two-component adhesive and a glass cap (4).

4. Process according to claims 1 to 3, characterized in that 0.1 to 100 femtolitres of injection substance are pressed within 5 minutes into the plastids, bacterium or cell compartment/cell nucleus.

5. Process according to claims 1 to 4, characterized in that the heat-expandable substance or substance mixture is a non-toxic, pure metal or metal alloy with a melting point between -20 and +200°C, which has an expansion coefficient comparable with mercury.

6. Process according to claim 5, characterized in that the non-toxic metal alloy is a gallium- indium-tin alloy, particularly Galinstan (trademark of Geraberger Thermometerwerke, Geschwenda, DE), which is liquid down to -20°C and has a volume expansion coefficient of 11.5γ 10⁻⁵ K⁻¹.

7. Process according to claim 6, characterized in that Galinstan is used together with a silicone oil.

8. Process according to claim 7, characterized in that four parts of Galinstan and one part of silicon oil are used for a borosilicate glass pipette.

9. Nanopipette (10) for performing the process according to one or more of the preceding claims, characterized by an external diameter of 0.5 to 2 mm, an internal diameter of 0.1 to 1.5 mm and a tip diameter of 0.025 to 0.3 µm, which is heatable by an adjustable heater (12) for temperature-controlled injection purposes.

## Revendications

1. Procédé de microinjection pour introduire une matière d'injection, en particulier une matière génétique étrangère, dans des cellules procaryotes et eucaryotes ainsi que des compartiments de cellules de ces'dernières (plastides, noyaux de cellules) au moyen d'une nanopipette et par pression,
caractérisé en ce
que l'on remplit la nanopipette (10) qui possède un diamètre extérieur de 0,5 à 2 mm, un diamètre intérieur de 0,1 à 1,5 mm et un diamètre de la pointe de 0,025 à 0,3 µm, avec la matière d'injection (1) et une matière ou un mélange expansible par la chaleur et que l'on ferme ensuite le tube capillaire de la nanopipette (10) avec une substance adhésive, que l'on pique la pointe de la pipette à l'aide d'un microscope et d'un micromanipulateur dans les plastides souhaités, les bactéries ou le compartiment de cellule/noyau de cellule et que l'on réchauffe la nanopipette au moyen d'un dispositif de chauffage réglable, jusqu'à ce que la matière d'injection sorte avec une vitesse d'écolement de l'ordre maximal d'1 femtolitre par seconde à la pointe de la pipette et entre dans les plastides, les bactéries ou le compartiment de cellule/le noyau de cellule dont le diamètre est de l'ordre d'1 à 20 µm.

2. Procédé de microinjection selon la revendication 1, caractérisé en ce qu'un courant d'air produit par une pompe (5) est soufflé par un tuyau (6) et un tube capillaire en verre (7) sur la nanopipette (10), l'air étant réchauffé par un tube en constantan (8) enroulé autour du tube capillaire en verre (7) qui est relié à un bloc d'alimentation (9) réglable en continu.

3. Procédé selon la revendication 1 ou 2, caractérisé en ce que la nanopipette (10) est fermée par une colle à deux composants et un capuchon en verre (4).

4. Procédé selon les revendications 1 à 3, caractérisé en ce que 0,1 à 100 femtolitres de matière d'injection est pressé en 5 minutes dans les plastides, les bactéries ou le compartiment de cellule/noyau de cellule.

5. Procédé selon les revendications 1 à 4, caractérisé en ce que la matière ou le mélange expansible à la chaleur est un métal pur non toxique ou un alliage de métal non toxique avec un point de fusion entre -20° et +200°C qui possède un coefficient de dilatation comparable à celui du mercure.

6. Procédé selon la revendication 5, caractérisé en ce que l'alliage de métal non toxique est un alliage de Galium-indium-étain, en particulier du Galinstan (marque des Geraberger Thermometerwerke, Geschwenda, DE) qui est liquide jusqu'à - 20° C et qui possède un coefficient de dilatation spatiale de 11,5 γ 10⁻⁵K⁻¹.

7. Procédé selon la revendication 6, caractérisé en ce que le Galinstan est utilisé ensemble avec une huile de silicone.

8. Procédé selon la revendication 7, caractérisé en ce que, pour une pipette de verre de borosilicate, on utilise 4 parties de Galinstan et 1 partie d'huile de silicone.

9. Nanopipette (10) pour exécuter le procédé selon l'une ou plusieurs des revendications précédentes, caractérisée par les caractéristiques suivantes : un diamètre extérieur de 0,5 à 2 mm, un diamètre intérieur de 0,1 à 1,5 mm et un diamètre de la pointe de 0,025 à 0,3 µm qui peut être réchauffée par une dispositif de chauffage réglable (12) pour l'injection thermocommandée.
